Europäisches Patentamt

European Patent Office

Office européen des brevets

(11) Publication number: **0 087 863**
**B1**

(12) **EUROPEAN PATENT SPECIFICATION**

(45) Date of publication of patent specification: **21.05.86**

(21) Application number: **83300620.8**

(22) Date of filing: **08.02.83**

(51) Int. Cl.⁴: **A 61 K 31/415,** A 61 K 31/20, A 61 K 31/23 // (A61K31/415, 31:23, 31:20)

(54) **Pharmaceutical and dietary composition.**

(30) Priority: **01.03.82 GB 8205936**

(43) Date of publication of application:
**07.09.83 Bulletin 83/36**

(45) Publication of the grant of the patent:
**21.05.86 Bulletin 86/21**

(84) Designated Contracting States:
**AT BE CH DE FR GB IT LI LU NL SE**

(56) References cited:
**BE-A- 890 888**
**FR-A-2 154 506**
**GB-A-1 082 624**

**Food compositions and nutrition tables 1981/82, Gibson and Kneebone, Am. J. Clinical Nutrition, vol. 34, p. 252-57 (1981)**

(73) Proprietor: **EFAMOL LIMITED**
**71/74 Mark Lane**
**London E.C.3 (GB)**

(72) Inventor: **Horrobin, David Frederick**
**P.O. Box 10**
**Nuns' Island Montreal H3E 1J8 (CA)**

(74) Representative: **Caro, William Egerton et al**
**J. MILLER & CO. Lincoln House 296-302 High Holborn**
**London WC1V 7JH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

### Field of Invention

The invention relates to the treatment of inflammatory disorders, including in particular but not exclusively rheumatoid arthritis.

### Background

The inventor does not limit himself by the following background and theory, but gives it to assist understanding.

In patients with rheumatoid arthritis is has recently been shown by a group in Montreal that levels of dihomo-γ-linolenic acid (DGLA) in blood phospholipids are exceptionally high. The work is that of Bruderlein et al, reported at the Golden Jubilee Congress on Essential Fatty Acids, Minneapolis, U.S.A., May 1980. The reason is unknown but the consequence is that in these patients DGLA is removed into complexed form and is unavailable for synthesis of prostaglandins (PG's), in particular 1-series prostaglandins including PGE1.

A great deal of interest has centered on PG metabolism in recent years, the outline of which from the normal dietary source linoleic acid by way of γ-linolenic acid (GLA) is as follows:—

cis-Linoleic Acid
(9,12-octadecadienoic acid)
↓
GLA
(6,9,12-octadecatrienoic acid)
↓

DGLA
ester ⇌ DGLA
reserves (8,11,14)-eicosatrienoic acid) → 1 series
(small) ↓ endoperoxides
↘
Large AA 1 series
AA ester ⇌ (Arachidonic acid i.e. P.G's
reserves 5,8,11,14-eicosatetraeonic acid)
↓
2 series
endoperoxides
↓
2 Series PG's

The broad outline of this pathway is well known, and it brings out clearly that a major function of essential fatty acids (EFAs) is to act as precursors for prostaglandins, 1-series PGs being formed direct from DGLA and 2-series PGs from arachidonic acid (AA). DGLA and AA are present in food in only small quantities, and the major EFA in food in linoleic acid which is first converted to γ-linolenic acid (GLA) and then to DGLA and AA, the latter step being irreversible. The conversion of linoleic acid to GLA is a limiting step, adequate in the young and healthy body but often inadequate in ageing or in many disease states.

DGLA is the key substance. GLA is almost completely and very rapidly converted in the body to DGLA and so for practical purposes the oral administration of DGLA and GLA amounts to the same thing. DGLA can be converted to a storage form, changed to arachidonic acid and thence to PGs of the 2-series, or converted to PGs of the 1-series.

A balance between 1-series and 2-series PGs is, the inventor believes, significant in terms of overall control of the conversion pathways given above. Such control is not understood in detail but without restriction to the theory it appears first that PGE2 is able to enhance the formation of 1-series PGs, and second that PGE1 is able to block arachidonic acid mobilisation from tissue stores. Thus the conditions for a negative feedback control loop exist; overproduction of PGE2 from AA will activate PGE1 synthesis, the PGE1 will inhibit AA mobilisation, and production of 2-series PGs will drop. Further, TXA2, an unstable product of the 2-series endoperoxides arising in 2-series PG production, also appears to limit arachidonic acid mobilisation and to enhance 1-series PG and in particular PGE1 production. Thus again the activity of the 2-series PG synthesis pathway gives rise indirectly to a material that controls the pathway.

There is increasing evidence that PGs of the 1-series play a vital role in a number of key areas. First, PGE1 activates T-lymphocytes. Defective T-lymphocytes are believed to be involved in causing a wide range of inflammatory disorders. Second, PGE1 is important in preventing overproduction of collagen and fibrous tissue, a factor which plays a major role in rheumatoid arthritis and the so-called collagen diseases. Third, PGE1 appears to inhibit the production of 2-series PGs, levels of which are raised in a wide variety of inflammatory disorders. Fourth, PGE1 increases production of cyclic AMP, which has anti-inflammatory effects.

There is a great deal of evidence that inflammation, including that found in rheumatoid arthritis, may be controlled by increasing the production of PGE1. Conversely inflammation may be made more likely by anything that tends to reduce the formation of PGE1.

In particular, it has recently been found that a critical factor in some inflammatory disorders, e.g. in the damage of myelin which occurs in multiple sclerosis, may be the entry of calcium into cells. This may damage mitochondria and activate destructive lysosomal enzymes.

Thus, there is now evidence which indicates that the regulation of the immune response and also the control of intracellular calcium may be significant factors in the treatment of various inflammatory disorders, e.g. rheumatoid arthritis, multiple sclerosis, Crohn's disease and other disorders listed below.

In inflammatory disorders, production of 2-series PGs from arachidonic acid is greatly exaggerated. These PGs are thought to contribute to the causation of the disease because steroids and aspirin-like drugs are both partially effective therapies, steroids blocking the conversion of AA esters to free AA and aspirin-like drugs blocking the conversion of free AA to endoperoxides which are intermediates in PG synthesis.

The overproduction of 2-series PGs implies that normal control of the PG synthetic pathway has been lost. Although control of this pathway is imperfectly understood two factors have been identified.

1. PGE1 is able to inhibit the formation of free AA from AA esters. This leads to the paradoxical fact that a partial EFA deficiency actually leads to increased formation of 2-series PGs, because DGLA stores are so much smaller than those of AA and a partial deficiency of EFAs will therefore lead to DGLA depletion first. This depletion will reduce formation of PGE1, remove the PGE1 control of AA and allow overproduction of 2-series PGs from the large AA stores.

2. An unstable product of AA metabolism, thromboxane A2 (TXA2) also feeds back to inhibit conversion of AA ester to free AA and possibly also of free AA to 2 series endoperoxides. Thus, loss of TXA2 will also lead to overproduction of 2-series PGs. TXA2 and PGE1 thus cooperate in the regulation of formation of 2-series PGs and a fault in the formation of either will lead to abnormalities.

Thus for example the disorders of PG synthesis in inflammatory disorders can be accounted for by inadequate formation of PGE1 and/or TXA2.

The evidence for direct involvement of PGs in inflammatory disorders has been briefly mentioned. There is also indirect evidence that PGs may act by regulating — or failing to regulate — the calcium movements into and out of cells already mentioned above. The calcium concentration in cytoplasm is normally very low and there is now excellent evidence from many sources that a brief rise in cytoplasmic calcium concentration triggers a variety of cell events, including activation of lysosomes which contain destructive enzymes. Normally this calcium is very rapidly removed after this brief activation so terminating the event. PGs and related substances have specific actions on calcium and the present inventor has obtained evidence to suggest that TXA2 and PCF2α is of critical importance. In particular, specific inhibition of TXA2 synthesis greatly prolongs the time taken for calcium to be removed from the cytoplasm after activation. Furthermore, inhibition of TXA2 synthesis leads to increased formation of PGF2α and PGE2 which can promote calcium entry into cells. There is thus good evidence that in this respect also PGE1 and TXA2 enhance one another's effects. In particular, in muscle the degree of contraction is related to the calcium concentration in the cytoplasm and muscle contraction is a measure of this calcium concentration. After inhibition of TXA2 synthesis the recovery from a contraction is greatly prolonged indicating slow removal of calcium. Further, inhibition of TXA2 synthesis can lead to a chronic state of partial contraction indicating the entry of calcium into the cytoplasm. PGF2α and PGE2 whose output is increased by inhibition of TXA2 synthesis also cause contraction indicating calcium entry into the cytoplasm.

Thus loss of TXA2 and PGE1 synthesis will lead to increased formation of 2-series PGs and entry of calcium into the cytoplasm. The inventor believes that this calcium activates lysosomes whose destructive enzymes play a large part in inflammation.

On general grounds there are therefore reasons to suppose that suppression of excess production of 2-series PGs will have desirable effects in inflammatory disorders.

Currently available conventional methods of suppression are administration of steroids and aspirin®-like drugs. However, while these may suppress overproduction of 2-series PGs they will exaggerate further any deficiencies in PGs of the 1-series and in TXA2, which may explain why they control symptoms but do not usually alter the long term course of the disease.

The present invention, the use of biotin as discussed below, controls excess PGE2 series production by restoring towards normal, or enhancing, the formation of 1-series PGs or, broadly, influencing the 1-series/2-series PG balance in the body in favour of 1-series PGs.

### Biotin

The inventor has been led to his present proposal by an isolated report at a meeting of the American Oil Chemists Society in Chicago in December 1981, where R. Holman reported an observation that a child with biotin deficiency has a very high level of DGLA in plasma phospholipids. Administration of biotin, among other and more obvious effects from reversing the deficiency, restored DGLA in phospholipids to normal. The similarity in the plasma phospholipids levels to that seen in rheumatoid arthritis struck the inventor, and suggested to him that administration of biotin to patients with rheumatoid arthritis and other inflammatory disorders will be beneficial. Instead of being bound in phospholipids, DGLA is free, allowing its conversion to PGE1 and thus indirectly exerting an anti-inflammatory effect. It is also the case that along with numerous other materials human milk contains γ-linolenic acid and biotin (Gibson & Kneebone, Am. J. Clinical Nutrition *34* 252—257 (1981); Food Composition and Nutrition Tables 1981/82 (Wissenschaftliche Verlagsgesellschaft, Stuttgart) pages 1 to 5, "Mother's Milk"). Such prior material is seen as only formally relevant.

## The Invention

The invention is to provide compositions of biotin to treat inflammatory disorders (dose 100 μg to 1 g/day, preferably 100 μg to 10 mg/day), in conjunction with enhanced supplies of essential fatty acids which are specifically, in view of the limited conversion of linoleic acid to GLA in the body, GLA 0.05 to 10 g/day or molar equivalent of DGLA or derivatives as discussed later herein.

The invention as above may further be used in conjunction with the inventor's previous proposals for selectively enhancing 1-series PG production or, more broadly expressed, for influencing the 1-series/2-series PG balance in the body in favour of 1-series PGs. These proposals include use of zinc, β-lactam antibiotics and other materials listed and discussed in published European Patent Specification No. A 0 003 407; use of penicillamine/phenformin and levamisole, and colchicine, Vinca alkaloids and other materials, listed and discussed in published European Patent Specification No. A 0 004 770; use of vitamin C, ethanol and opiate antagonists listed and discussed in published European Patent Specification No. A 0 019 423; and use of 4-amino and 8-amino quinolines, acridines, quinine and other materials including spironolactone listed and discussed in published European Patent Specification No. A 0 035 856. Reference may be made to these specifications for the full listings, discussion and dosages, which are applicable in the present context also.

## Inflammatory Disorders Treated

The disorders that can be treated include multiple sclerosis, systemic lupus erythematosus; Crohn's disease; ulcerative colitis; inflammatory diseases of the kidney, for example glomerulonephritis and nephrotic syndrome; inflammatory and degenerative diseases of the nervous system and muscular systems, for example, muscular dystrophies, Friedreich's ataxia and related conditions of peripheral nerve degeneration; disorders of an auto-immune nature, and other collagen related diseases; rheumatoid arthritis and other inflammatory joint disorders; inflammatory skin disorders; disorders characterised by recurrent inflammation such as Familial Mediterranean Fever or Behcet's syndrome.

## Packs

If it is not desired to have compositions comprising active materials listed above, packs may be prepared comprising the materials presented for separate or part joint and part separate administration in the appropriate relative amounts, and such packs are within the purview of the invention.

## Dietary Compositions

The invention is chiefly described in terms of use of pharmaceutical compositions, but it will be understood that the γ-linolenic and other acids being in the nature of dietary supplements, could be incorporated in a dietary margarine or other foodstuffs; use of such foodstuffs, possibly containing other active materials and generally referred to in this description as dietary or pharmaceutical compositions, are within the purview of the invention and thus of the term pharmaceutical compositions, packs or the like used in the claims.

## Veterinary Applications

It will be understood that where a disorder of a kind calling for treatment in animals arises, the invention while described primarily in terms of human medicine and treatment is equally applicable in the veterinary field.

## Amounts of γ-linolenic and Other Acids Specifically

A preferred daily dosage for inflammatory disorders for an adult (weight ca 75 kg) is from 0.05 to 0.1 up to 1, 2, 5 or even 10 g as required of γ-linolenic acid or equivalent weight (calculated as γ-linolenic acid) or physiologically functional ester, salt or other derivative thereof. Amounts may in particular be 0.1 to 1.0 g daily. Such doses correspond to about 2 to 20 g daily of Oenothera oil discussed below. In place of, or in addition to, γ-linolenic acid, one may use dihomo-γ-linolenic acid or a physiologically functional ester, salt or other derivative thereof, in amounts equivalent in molar terms to γ-linolenic acid and calculated as such. Other EFA's are likewise related back to γ-linolenic acid in molar terms. The dosage can for example be taken as a single dose or divided into 2, 3 or 4 subdivisions thereof as convenient.

Again based on present evidence, a particularly suitable daily dosage in inflammatory disorders for an adult (weight ca 75 kg) would be from 0.1 to 1.0 g of γ-linolenic acid or equivalent weight of derivative thereof. Linolenic acid, if alternatively given, may be in amounts of, for example, 1 to 100 g, preferably 3 to 10 g per day.

## Forms and Source of γ-linolenic and Other Acids Suitable physiologically functional derivatives, convertible in the body to GLA or DGLA to enter the biosynthetic pathway given earlier herein, are physiologically acceptable salts, esters (particularly glycerides and simple $C_1$—$C_4$ alkyl esters), amides and phospholipids. Indirect indentification of useful derivatives is by their having the valuable effect in the body of the acid (GLA or DGLA) itself, but conversion can be shown directly by gas chromatographic analysis of GLA or DGLA concentration in blood, body fat, or other tissue by standard techniques for example those of Pelick et al. p. 23, "Analysis of Lipids and Lipoproteins" Ed. Perkins, American Oil Chemists Society, Champaign, Illinois, U.S.A.

If desired, pharmaceutical compositions may be produced for use in the invention by associating natural or synthetic γ-linolenic acid (or a physiologically functional derivative thereof) and/or dihomo-γ-linolenic acid (or a physiologically functional derivative thereof) as such, with an acceptable pharmaceutical vehicle. It is at present convenient to incorporate the γ-linolenic acid into compositions in the form of an available oil having a high γ-linolenic acid content.

At the present time known natural sources of oils having a high γ-linolenic acid content are few (there are no known natural sources of significant amounts of dihomo-γ-linolenic acid). One source of oils currently available is the seed of Evening Primrose species such as *Oenothera biennis L.* and *Oenothera lamarckiana*, the oil extract therefrom containing γ-linolenic acid (about 8%) and linoleic acid (about 72%) in the form of their glycerides together with other glycerides (percentages based on total fatty acids). Another source of γ-linolenic acid is the seed of Borage species such as *Borago officinalis* which, though its current yield per acre is low, provides a richer source of γ-linolenic acid than Oenothera oil. Recent studies on fungi which can be cultivated by fermentation promise a fungal oil source.

The seed oil extracts referred to above can be used as such or can for example if desired be fractionated to yield an oily composition containing the triglycerides of γ-linolenic acid and linoleic acid as the main fatty acid components, the γ-linolenic acid content being if desired a major proportion. Seed oil extracts appear to have a stabilising effect upon any dihomo-γ-linolenic acid or physiologically functional derivative thereof incorporated therein.

Pharmaceutical Presentation

The compositions used according to the invention are conveniently in a form suitable for oral, rectal, parenteral or topical administration in a suitable pharmaceutical vehicle, as discussed in detail for example in U.K. Patent Specification No. 1 082 264 and in any case very well known generally for any particular kind of preparation. Thus for example, tablets, capsules, ingestible liquid or powder preparations, creams and lotions for topical application, or suppositories, can be prepared as required. Injectable solutions of hydrolysed Oenothera oil may be prepared using albumin to solubilise the free acid.

Advantageously a preservative is incorporated into the preparation. α-Tocopherol in a concentration of about 0.1% by weight has been found suitable for the purpose.

It will be understood that the absolute quantity of active ingredients present in any dosage unit should not exceed that appropriate to the rate and manner of administration to be employed but on the other hand should also desirably be adequate to allow the desired rate of administration to be achieved by a small number of doses. The rate of administration will moreover depend on the precise pharmacological action desired.

The following Examples serve to illustrate pharmaceutical compositions useful in treatment according to the invention:—

Examples

Pharmaceutical compositions containing a unit dose of an oil extract from the seeds of *Oenothera biennis L.* optionally with methyl dihomo-γ-linolenate and/or any of the other active materials referred to herein are prepared by encapsulation of the natural oil in soft gelatine capsules manufactured by known methods.

The oil is extracted from the seeds by one of the conventional methods of extraction such as cold pressure, screw pressure after partially cooking the seed, or solvent extraction.

Fractionation of a typical sample of this oil shows a yield of 97.0% oil in the form of methyl esters, with the relative proportions:

| | |
|---|---|
| Palmitate | 6.15 |
| Stearate | 1.6 |
| Oleate | 10.15 |
| Linoleate | 72.6 |
| γ-Linolenate | 8.9 |

As preservative, α-tocopherol is added to the oil in a concentration of 0.1%.

Gelatin capsules containing oil extracts prepared as described above, each having the following contents of active ingredients (0.5 g oil extract = ca 0.045 g γ-linolenic acid) are prepared in conventional fashion.

Example 1

| | |
|---|---|
| Oil extract | 0.5g |
| Biotin | 0.5 mg |

Two capsules may be administered thrice daily in the treatment of inflammatory disorders as above, giving a daily dose of γ-linolenic acid of ca. 0.27 g.

Example 2

| | |
|---|---|
| Oil extract | 0.5 g |
| Methyl dihomo-γ-linolenate | 10 mg |
| Biotin | 0.5 mg |

Two capsules may be administered thrice daily in the treatment of inflammatory disorders as above.

**Claims for the Contracting States: BE CH DE FR GB IT LI LU NL SE**

1. A pharmaceutical or dietary composition comprising one or more essentially fatty acids in conjunction with biotin; the or each essential fatty acid being γ-linolenic acid or dihomo-γ-linolenic acid or physiologically functional salt, ester or other derivative convertible in the body thereto, and the composition being presented for administration to give 0.05 to 10 g γ-linolenic acid daily or molar equivalent of dihomo-γ-linolenic acid or derivative and 100 µg to 1 g biotin daily.

2. A composition according to claim 1, wherein said amount of biotin is 100 µg 10 mg daily.

3. A pharmaceutical pack comprising the materials set out in either preceding claim presented separately, or one or more separately and others together, but for joint administration.

4. The composition of claim 1 or 2 or the pack of claim 3, for use in treatment of inflammatory disorders.

**Claims for the Contracting State: AT**

1. The use of γ-linolenic acid or dihomo-γ-linolenic acid or physiologically functional salt, ester or other derivative convertible in the body thereto, together with biotin, alone or in conjunction with an acceptable pharmaceutical or dietary vehicle for the method of producing a pharmaceutical or dietary composition for treatment of inflammatory disorders, said composition being presented for administration to give 0.5 to 10 g γ-linolenic acid daily or molar equivalent of dihomo-γ-linolenic acid or derivative and 100 µg to 1 g biotin daily.

2. The use claimed in claim 1, said composition being presented to give 100 µg to 10 mg biotin daily.

**Patentansprüche für die Vertragsstaaten: BE CH DE FR GB IT LI LU NL SE**

1. Pharmazeutisches oder Nahrungsmittel-Präparat aus mindestens einer essentiellen Fettsäure in Verbindung mit Biotin, wobei die essentielle Fettsäure oder die essentiellen Fettsäuren γ-Linolensäure oder Dihomo-γ-Linolensäure oder ein im Körper dazu unwandelbares Salz, umwandelbarer Ester oder umwandelbares sonstiges Derivat, ist (sind), und wobei das Präparat dergestalt zur Verabreichung dargeboten wird, daß 0,05 bis 10 g γ-Linolensäure pro Tag oder das molare Äquivalent von Dihomo-γ-Linolensäure oder Derivat sowie 100 µg bis 1 g Biotin pro Tag verabfolgt werden.

2. Präparat nach Anspruch 1, dadurch gekennzeichnet, daß die Biotinmenge 100 µg bis 10 mg pro Tag beträgt.

3. Pharmazeutische Packung, mit der die in einem der vorhergehenden Ansprüche angegebenen Substanzen getrennt oder mindestens eine Substanz getrennt und andere in Verbindung miteinander, aber zu gemeinsamer Verabreichung, dargeboten werden.

4. Präparat nach Anspruch 1 oder 2, oder Packung nach Anspruch 3 zur Verwendung bei der Behandlung von Entzündungsvorgängen.

**Patentansprüche für den Vertragsstaat: AT**

1. Verwendung von γ-Linolensäure oder Dihomo-γ-Linolensäure oder einem in Körper dazu unwandelbaren Salz, Ester oder sonstigen Derivat zusammen mit Biotin für sich allein oder in Verbindung mit einem zulässigen pharmazeutischen oder Nahrungsmittel-Träger für das Verfahren zur Herstellung eines pharmazeutischen oder Nahrungsmittel-Präparats für die Behandlung von Entzündungsvorgängen, wobei das Präparat dergestalt zur Verabreichung dargeboten wird, daß 0,05 bis 10 g γ-Linolensäure pro Tag oder das molare Äquivalent von Dihomo-γ-Linolensäure oder eines Derivats und 100 µg bis 1 g Biotin pro Tag verabfolgt werden.

2. Verwendung nach Anspruch 1, wobei das Präparat dergestalt dargeboten wird, daß 100 µg bis 10 mg Biotin pro Tag verabfolgt werden.

**Revendications pour les Etats contractants: BE CH DE FR GB IT LI LU NL SE**

1. Composition pharmaceutiques ou diététiques comprenant un ou plusieurs acides gras essentiels conjointement avec de la biotine; le ou chaque acide gras essentiel étant l'acide γ-linolénique ou l'acide dihomo-γ-linolénique ou un de ses sels esters ou autres dérivés physiologiquement fonctionnels transformables dans l'organisme en l'acide, et la composition étant présentée pour l'administration pour donner 0,05 à 10 g d'acide γ-linolénique par jour ou l'équivalent molaire d'acide dihomo-γ-linolénique ou de son dérivé et 100 µg à 1 g de biotine par jour.

2. Composition selon la revendication 1, dans laquelle ladite quantité de boitine est de 100 µg à 10 mg par jour.

3. Conditionnement pharmaceutique comprenant les substances indiquées dans l'une quelconque des revendications précédentes, présentées séparément ou une ou plus séparément et les autres ensemble, mais pour l'administration conjointe.

4. Composition selon la revendication 1 ou 2 ou conditionnement selon la revendication 3, pour l'utilisation dans le traitement des troubles inflammatoires.

**Revendications pour l'Etat contractant: AT**

1. Utilisation de l'acide γ-linolénique ou de l'acide dihomo-γ-linolénique ou d'un sel, ester ou autres dérivés physiologiquement fonctionnels pouvant être transformés dans l'organisme en l'acide, conjointement avec la biotine, seuls ou avec un véhicule acceptable en pharmacie ou en diététique, pour le procédé de production d'une composition pharmaceutique ou diététique pour le traitement des troubles inflammatoires, ladite composition étant présentée pour l'administration pour donner 0,05 à 10 g d'acide γ-linolénique par jour ou l'équivalent molaire d'acide dihomo-γ-linolénique ou son dérivé et 100 μg à 1 g de biotine par jour.

2. Utilisation selon la revendication 1, ladite composition étant présentée pour donner 100 μg à 10 mg de biotine par jour.